(19) Europäisches Patentamt
European Patent Office
Office européen des brevets

(11) Veröffentlichungsnummer: **0 425 930 B1**

(12) **EUROPÄISCHE PATENTSCHRIFT**

(45) Veröffentlichungstag der Patentschrift: **28.09.94**

(21) Anmeldenummer: **90120077.4**

(22) Anmeldetag: **19.10.90**

(51) Int. Cl.5: **C07C 67/343**, C07C 69/76, C07C 45/72, C07C 49/813, C07C 49/796, C07C 317/14, C07C 315/04

# (54) Verfahren zur Herstellung von symmetrischen Diarylacetylenen.

(30) Priorität: **01.11.89 DE 3936297**

(43) Veröffentlichungstag der Anmeldung:
**08.05.91 Patentblatt 91/19**

(45) Bekanntmachung des Hinweises auf die Patenterteilung:
**28.09.94 Patentblatt 94/39**

(84) Benannte Vertragsstaaten:
**BE CH DE FR GB IT LI**

(56) Entgegenhaltungen:
**EP-A- 0 050 428**

(73) Patentinhaber: **BAYER AG**

**D-51368 Leverkusen (DE)**

(72) Erfinder: **Bader, Axel, Dr.**
**Heymannstrasse 40**
**W-5090 Leverkusen 1 (DE)**
Erfinder: **Arlt, Dieter, Prof. Dr.**
**Rybniker Strasse 2**
**W-5000 Köln 80 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft ein Verfahren zur Herstellung von symmetrischen Diaryl-acetylenen aus Arylbromiden und Acetylen in Gegenwart eines Palladium-Katalysators und einer Base, wobei man das Acetylen mit Hilfe einer Intensivbegasungseinrichtung in das flüssige Reaktionsgemisch einleitet.

Symmetrische Diaryl-acetylene sind wertvolle Zwischenprodukte. Beispielsweise können solche Diaryl-acetylene die an den Arylresten je eine Carboxyl-, Carbonsäureester- oder Carbonsäurehalogenidgruppe tragen, oder eine Gruppe, die in eine Carboxylgruppe, eine Estergruppe oder ein Carbonsäurehalogenid umgewandelt werden kann, an der Dreifachbindung partiell hydriert werden, und das so gebildete bifunktionelle Diaryl-olefin kann durch Polykondensation zu einem hochwärmefesten Chemiewerkstoff umgesetzt werden.

Diaryl-acetylene lassen sich beispielsweise durch Dehydrohalogenierung von Diaryl-halogenalkenen bzw. durch zweifache Dehydrohalogenierung von vicinalen oder geminalen Diaryl-dihalogenalkanen mit starken Basen unter relativ drastischen Reaktionsbedingungen herstellen (Houben-Weyl, Methoden der Organischen Chemie, Band V/2a (1977), S, 53 ff.). Diese Reaktionen sind nicht geeignet für die Herstellung von Diaryl-acetylenen mit basenempfindlichen Gruppen. Darüber hinaus sind die als Ausgangsmaterial verwendeten Halogenverbindungen oft schlecht zugänglich.

Eine weitere Methode zur Herstellung symmetrischer Diaryl-acetylene ist die Castro-Stephans-Reaktion (loc. cit., S 563 ff.). Hierin werden Kupferacetylide mit Aryliodiden in siedendem Pyridin zu disubstituierten Acetylenen umgesetzt. Zur Erzielung annehmbarer Ausbeuten ist dieses mehrstufige Verfahren auf die relativ teuren und oft schlecht zugänglichen Aryliodide begrenzt.

Weiterhin lassen sich Diaryl-acetylene, ausgehend von Aryliodiden oder Arylbromiden und Arylacetylenen unter Palladium-Katalyse, herstellen (Houben-Weyl, Band XIII/9b (1984), S. 987 ff). Die hierfür benötigten Arylacetylene sind jedoch nur sehr aufwendig in mehrstufigen Verfahren zugänglich.

Bekannt ist außerdem, das sich Diaryl-acetylene durch Umsetzung von Acetylen mit Halogenverbindungen in Gegenwart von Bis-(triphenylphosphin)-palladium-dichlorid und Kupfer(I)-iodid in Lösung von Diethylamin herstellen lassen (Tetrahedron Lett. (1975), 4467). Als Halogenverbindungen lassen sich hierbei Bromalkene oder Brompyridin einsetzen; in der Benzolreihe ist diese Reaktion auf Aryliodide begrenzt. Zur Durchführung wird in einen Ansatz von 10 mmol Iodbenzol durch ein Einleitungsrohr 6 Stunden lang Acetylen in die geführte Losung eingeleitet.

Es wurde nun überraschend gefunden, das es unter Anwendung einer intensiven Begasung des flüssigen Reaktionsgemisches mit Acetylen möglich ist, die stabileren, einfacher herstellbaren und damit billigeren Arylbromide anstelle der Aryliodide einzusetzen und gleichzeitig die Reaktion auch in größeren Ansatzen zu beschleunigen (größere Raum-Zeit-Ausbeute). Damit wird diese Reaktion auch für technische Anwendungen, beispielsweise für den obengenannten Einsatz, zugänglich.

Es wurde ein Verfahren zur Herstellung von symmetrischen Diaryl-acetylenen der Formel

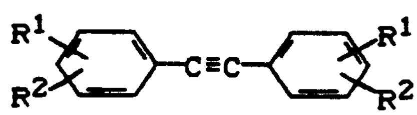

(I),

in der

| | |
|---|---|
| $R^1$ | Wasserstoff, geradkettiges oder verzweigtes $C_1$-$C_8$-Alkyl, $C_3$-$C_8$-Cycloalkyl, Phenyl, $C_7$-$C_{10}$-Aralkyl, geradkettiges oder verzweigtes $C_1$-$C_8$-Alkoxy, Phenoxy, Benzyloxy, $\beta$-Phenyl-ethenyl, Phenyl-ethinyl, Phenyl-sulfonyl, Phenyl-carbonyl, Hydroxy in freier oder geschützter Form, Fluor, Chlor, Nitro, Cyano, $COR^3$, $NR^5R^5$ oder $CONR^4R^5$ bedeutet und |
| $R^2$ | für Wasserstoff, geradkettiges oder verzweigtes $C_1$-$C_8$-Alkyl, Fluor, Chlor, Cyano, $COR^3$ oder $CONR^4R^5$ steht und |
| $R^1$ und $R^2$ | gemeinsam, wenn sie benachbart sind, eine Dicarbonsäureanhydridgruppe bilden können, wobei |
| $R^3$ | Wasserstoff, Hydroxy, geradkettiges oder verzweigtes $C_1$-$C_8$-Alkyl, geradkettiges oder verzweigtes $C_1$-$C_8$-Alkoxy, Phenyl oder Phenoxy bedeutet und |
| $R^4$ und $R^5$ | unabhängig voneinander für Wasserstoff oder geradkettiges oder verzweigtes $C_1$-$C_8$-Alkyl stehen und $R^4$ zusätzlich $C_2$-$C_4$-Alkoxycarbonyl oder $C_2$-$C_4$-Acyl sein kann, wobei weiterhin $R^4$ und $R^5$ gemeinsam $C_4$-$C_8$-Alkylen bilden können, |

und

wobei die Benzolkerne in den Resten $R^1$ und $R^2$ ihrerseits durch geradkettiges oder verzweigtes $C_1$-$C_8$-Alkyl, geradkettiges oder verzweigtes $C_1$-$C_8$-Alkoxy, Fluor, Chlor oder $COR^3$ substituiert sein können,

durch Umsetzung eines Arylhalogenids mit Acetylen in Gegenwart eines Palladium-Katalysators und einer Base gefunden, das dadurch gekennzeichnet ist, daß man das Acetylen mit Hilfe einer Intensivbegasungseinrichtung in das flüssige Reaktionsgemisch einleitet und als Arylhalogenide Arylbromide der Formel

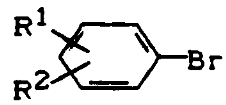

(II)

einsetzt, in der

$R^1$ und $R^2$ die obige Bedeutung haben.

Geradkettiges oder verzweigtes $C_1$-$C_8$-Alkyl ist beispielsweise Methyl, Ethyl, Propyl, Isopropyl, Butyl, Isobutyl, tert.-Butyl, die isomeren Amyle, Hexyle oder Octyle. Bevorzugt unter diesen sind die genannten $C_1$-$C_4$-Alkyle, besonders bevorzugt sind Methyl und Ethyl.

$C_3$-$C_8$-Cycloalkyl ist beispielsweise Cyclopropyl, Methylcyclopropyl, Dimethyl-cyclopropyl, Tetramethyl-cyclopropyl, Cyclobutyl, Cyclopentyl, Methyl-cyclopentyl, Cyclohexyl, Methyl-cyclohexyl, Cycloheptyl und Cyclooctyl. Bevorzugt unter diesen sind Cyclopropyl, Cyclopentyl und Cyclohexyl sowie ihre einfach oder mehrfach durch Methyl substituierten Derivate.

$C_7$-$C_{10}$-Aralkyl ist beispielsweise Benzyl, $\alpha$- oder $\beta$-Phenyl-ethyl, $\alpha$-, $\beta$- oder $\gamma$-Phenyl-propyl oder -isopropyl; bevorzugt unter diesen ist Benzyl.

Geradkettiges oder verzweigtes $C_1$-$C_8$-Alkoxy ist beispielsweise Methoxy, Ethoxy, Propoxy, Isopropoxy, Butoxy, Isobutoxy, tert.-Butoxy, die isomeren Amyle, Hexyle und Octyle. Bevorzugt unter diesen sind die genannten $C_1$-$C_4$-Alkoxy-Gruppen, besonders bevorzugt sind Methoxy und Ethoxy.

Hydroxy als $R^1$ kann in freier oder geschützter Form vorliegen. Als geschützte Form sei die veresterte oder veretherte Form genannt, aus der die freie Form wieder hergestellt werden kann. Als veresterte Form sei beispielsweise der Acetyl- oder Propionylester genannt; als veretherte Form sei beispielsweise der Tetrahydropyranylether, der Trialkylsilylether oder der tert.-Alkylether genannt.

Für den Fall, das die Reste $R^4$ und $R^5$ gemeinsam $C_4$-$C_8$-Alkylen bilden, sind die entsprechenden Amin-Substituenten von cyclischen Basen wie Pyrrolidin, Piperidin, Aza-cyclohexan oder Aza-cyclooctan, abgeleitet.

Als Arylbromide werden vorzugsweise solche der Formel

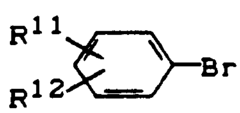

(III)

eingesetzt, in der

| | |
|---|---|
| $R^{11}$ | Wasserstoff, geradkettiges oder verzweigtes $C_1$-$C_8$-Alkyl, Phenyl, Benzyl, geradkettiges oder verzweigtes $C_1$-$C_8$-Alkoxy, Phenoxy, Benzyloxy, $\beta$-Phenylethenyl, Phenyl-sulfonyl, Phenyl-carbonyl, Fluor, Chlor, Nitro, Cyano, $COR^{13}$ oder $NR^{14}R^{15}$ bedeutet und |
| $R^{12}$ | für Wasserstoff, geradkettiges oder verzweigtes $C_1$-$C_8$-Alkyl, Fluor, Chlor, Cyano oder $COR^{13}$ steht und |
| $R^{11}$ und $R^{12}$ | gemeinsam, wenn sie benachbart sind, eine Dicarbonsäureanhydridgruppe bilden können, wobei |
| $R^{13}$ | Wasserstoff, Hydroxy, geradkettiges oder verzweigtes $C_1$-$C_4$-Alkyl oder geradkettiges oder verzweigtes $C_1$-$C_4$-Alkoxy bedeutet und |
| $R^{14}$ und $R^{15}$ | unabhängig voneinander für Wasserstoff oder geradkettiges oder verzweigtes $C_1$-$C_4$-Alkyl stehen und $R^{14}$ zusätzlich $C_2$-$C_4$-Acyl sein kann, wobei weiterhin $R^{14}$ und $R^{15}$ gemeinsam $C_4$-$C_8$-Alkylen bilden können, und |

wobei die Benzolkerne in den Resten $R^{11}$ und $R^{12}$ ihrerseits durch geradkettiges oder

verzweigtes $C_1$-$C_4$-Alkyl, geradkettiges oder verzweigtes $C_1$-$C_4$-Alkoxy, Fluor, Chlor oder $COR^{13}$ substituiert sein können.

Besonders bevorzugt werden als Arylbromide solche der Formel

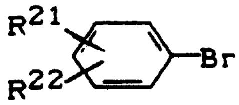

(IV)

eingesetzt, in der

$R^{21}$ Wasserstoff, geradkettiges oder verzweigtes $C_1$-$C_4$-Alkyl, Phenyl, β-Phenyl-ethenyl, Phenyl-sulfonyl, Phenyl-carbonyl, Fluor, Chlor, Cyano oder $COR^{23}$ bedeutet und

$R^{22}$ für Wasserstoff, Methyl, Ethyl, Cyano oder $COR^{23}$ steht und

$R^{21}$ und $R^{22}$ gemeinsam, wenn sie benachbart sind, eine Dicarbonsäureanhydridgruppe bilden können,

wobei

$R^{23}$ Wasserstoff, Hydroxy, Methyl, Ethyl, Methoxy oder Ethoxy bedeutet und wobei die Benzolkerne im Rest $R^{21}$ ihrerseits durch Fluor, Chlor oder $COR^{23}$ substituiert sein können.

Beispielhaft seien folgende Arylbromide für das erfindungsgemäße Verfahren aufgezählt: Brombenzol, 2-, 3-oder 4-Carboxy-brombenzol, 2-, 3- oder 4-Formyl-brombenzol, die verschiedenen isomeren Chlor-brombenzole, Fluor-brombenzole, Chlor-fluor-brombenzole, Dichlorbrombenzole, Difluor-brombenzole, Bromtoluole, Bromxylole, Brombenzol-dicarbonsäuren, Brombenzol-dicarbonsäuremethyl(propyl, butyl usw.)ester, 2-, 3- oder 4-Brombenzonitril, 3- oder 4-Bromphthalodinitril, 3- oder 4-Bromphthalsäureanhydrid, 3- oder 4-Brombenzoesäureethylester, 3-Methyl(oder Ethyl)-4-carboxybrombenzol, 3-Carboxy-4-methyl(oder ethyl)-brombenzol, 2-, 3- oder 4-Bromacetophenon, 3-Brom-6-methylacetophenon, 2-Methyl(oder Ethyl)-4-Bromacetophenon, 4-Brom-4′-fluordiphenylsulfon, 4-Brom-4′-chlordiphenylsulfon, 4-Brom-3′-fluordiphenylsulfon, 4-Brom-3′-chlordiphenylsulfon, 3-Brom-4′-fluordiphenylsulfon, 3-Brom-3′-fluordiphenylsulfon, 3-Brom-4′-chlordiphenylsulfon, 3-Brom-3′-chlordiphenylsulfon, 4-Brom-4′-carboalkoxy(methoxy, ethoxy usw.)stilbene, 4-Brom-3′-carboalkoxy(methoxy, ethoxy usw.)stilbene, 3-Brom-4′-carboalkoxystilben, 3-Brom-3′-carboalkoxystilbene, 4-Brom-4′-carboxystilben, 4-Brom-3′-carboxystilben, 3-Brom-4′-carboxystilben, 3-Brom-3′-carboxystilben, 4-Brom-4 -fluorbenzophenon, 4-Brom-4′-chlorbenzophenon, 4-Brom-3′-fluorbenzophenon, 4-Brom-3′-chlorbenzophenon, 3-Brom-4′-fluorbenzophenon, 3-Brom-4′-chlorbenzophenon, 3-Brom-3′-fluorbenzophenon, 3-Brom-3′-chlorbenzophenon, 4-Brom-4′-carboxybenzophenon, 4-Brom4′-carboalkoxy(methoxy, ethoxy usw.) benzophenone, 4-Brom-4′-carboxybiphenyl, 4-Brom-4′-carboalkoxy(methoxy, ethoxy usw.)biphenyle, 4-Acetyl-4′-brombiphenyl, 4-Acetyl-3′-brombiphenyl.

Das erfindungsgemäße Verfahren wird in Gegenwart eines Palladium-Katalysators durchgeführt. Dieser Palladium-Katalysator kann auch aus einem Gemisch mehrerer der unten aufgeführten Palladium-Verbindungen bestehen. In bevorzugter Weise, vor allem wegen der leichteren Aufarbeitung und Wiederverwendung, wird jedoch nur eine der genannten Palladium-Verbindungen eingesetzt. Als solche Palladium-Katalysatoren kommen beispielsweise folgende in Frage: Palladium-Schwarz, Palladium auf Kohle, einfache anorganische und organische Palladium-Salze, komplexierte anorganische und organische Palladium-Salze, wobei als Komplexbildner Nitrile, wie Benzonitril oder ein $C_1$-$C_4$-Alkylnitril, wie Acetonitril, Triphenylarsin oder ein unten naher charakterisiertes Phosphin fungieren, ferner Phenyl-palladium-chlorid(bromid,iodid)-diphosphin oder Palladium-tetraphosphine, wobei in diesen Fällen ebenfalls das unten näher charakterisierte Phosphin auftritt, ferner Di-(dibenzyliden-aceton)-palladium (= $(dbe)_2Pd$) oder Di-(1,1-bis[dibenzylphosphin]-ferrocen)-palladiumdichlorid, -dibromid oder -diiodid (= $(dppf)_2PdCl_2(Br_2,I_2)$).

Einfache anorganische und organische Palladium-Salze sind beispielsweise das Chlorid, das Bromid, das Iodid, das Nitrat, das Sulfat, das Acetat, das Propionat, bevorzugt die genannten Halogenide und das Acetat. Diese Salze können auch mit den genannten Komplexbildnern komplexiert sein, wobei im Falle der Phosphine solche der Formel

$$P(R^6R^7R^8) \qquad (V)$$

genannt seien, in der

$R^6$, $R^7$ und $R^8$ unabhängig voneinander geradkettiges oder verzweigtes $C_1$-$C_4$-Alkyl oder Phenyl ist, wobei Phenyl durch geradkettiges oder verzweigtes $C_1$-$C_4$-Alkyloder $C_1$-$C_4$-Alkoxy

oder durch Fluor, Chlor oder Brom substituiert sein kann.

Die genannten Palladium-Verbindungen lassen sich formelmäßig wie folgt darstellen: Pd, Pd-Schwarz, Pd/C, $PdX_2$ (X = obengenanntes Anion), $(C_6H_5\text{-}CN)_2PdX_2$, $(C_1\text{-}C_4\text{-}CN)_2PdX_2$, $(As[C_6H_5]_3)PdX_2$, $(PR^6R^7R^8)_2PdX_2$, $C_6H_5PdI(PR^6R^7R^8)_2$, $Pd(PR^6R^7R^8)_4$, $(dbe)_2Pd$ und $(dppf)_2PdHal_2$, Bevorzugte Palladium-Katalysatoren sind hierbei die genannten organischen Palladium-Salze, die komplexierten anorganischen und organischen Palladium-Salze, bei denen ein Phosphin der genannten Art als Komplexbildner fungiert und Palladium-tetraphosphin mit einem Phosphin der genannten Art.

Die Aufzählung der genannten Palladium-Verbindungen ist nur unvollständig. Weitere Palladium-Verbindungen können vom Fachmann durch einfaches Ausprobieren ebenfalls im erfindungsgemäßen Verfahren eingesetzt werden. Alle diese Palladium-Verbindungen sind dem Fachmann bekannt.

Der Palladium-Katalysator (eine oder mehrere Pd-Verbindungen) wird in einer Menge von 0,0005-5 Mol-%, bevorzugt 0,05-2 Mol-%, bezogen auf das Arylbromid, eingesetzt.

Es kann wünschenswert sein und ist daher eine bevorzugte Variante des erfindungsgemäßen Verfahrens, den Palladium-Katalysator durch Zusatz eines oder mehrerer Phosphine der Formel (V) zu aktivieren, wobei ein solches Phosphin in einer Menge von 100-2000 Mol-%, bezogen auf den eingesetzten Palladium-Katalysator, eingesetzt wird.

Es kann weiterhin wünschenswert sein und ist eine weitere bevorzugte Variante des erfindungsgemäßen Verfahrens, dem Palladium-Katalysator ein oder mehrere Kupfersalz(e) als Co-Katalysator in einer Menge von 50-1000 Mol-%, bezogen auf den Palladium-Katalysator, zuzusetzen. Als solche Kupfersalze kommen bevorzugt Kupferhalogenide (Chloride, Bromide oder Iodide von Cu(I) oder Cu(II)) oder Kupferacetat in Frage. In besonders bevorzugter Weise wird Cu(I)-Iodid oder Cu(II)-acetat-Monohydrat eingesetzt.

Als Base für das erfindungsgemäße Verfahren kommt eine große Anzahl dem Fachmann bekannter Verbindungen in Frage. Diese Verbindungen können grundsätzlich einzeln oder als Gemisch mehrerer von ihnen eingesetzt werden; aus Gründen der vereinfachten Reaktionsführung, Aufarbeitung und gegebenenfalls Recyclisierung wird bevorzugt nur eine der basischen Verbindungen eingesetzt. Beispielhaft seien genannt: die Hydride, Hydroxide, Oxide oder Amide der Alkalimetalle oder Erdalkalimetalle. Als (Erd)-Alkalimetalle kommen hierbei in Frage: Lithium, Natrium, Kalium, Rubidium, Cäsium, Magnesium, Calcium, Strontium oder Barium, bevorzugt Lithium, Natrium, Kalium oder Calcium. Als Basen kommen weiterhin in Frage die geradkettigen oder verzweigten $C_1$-$C_8$-Alkoholate der genannten Alkalimetalle oder Erdalkalimetalle, wobei der $C_1$-$C_8$-Alkoxyrest dem oben näher beschriebenen entspricht. Als basische Verbindungen kommen weiterhin Stickstoffbasen der Formel

$N(R^9R^{10}R^{16})$ (VI)

in Frage, in der

$R^9$ und $R^{10}$ unabhängig voneinander geradkettiges oder verzeigtes $C_1$-$C_8$-Alkyl oder beide gemeinsam $C_4$-$C_8$-Alkylen bedeuten, wobei in der Alkylenkette ein C-Atom durch ein O-Atom oder die Gruppe $-NR^{16}-$ ersetzt sein kann, und

$R^{16}$ Wasserstoff oder geradkettiges oder verzweigtes $C_1$-$C_4$-Alkyl darstellt.

Weiterhin kommen als Basen bicyclische Stickstoffverbindungen in Frage, wie beispielsweise Triethylendiamin (Diaza-bicyclo-octan = DABCO), Diaza-bicyclo-nonen (DBN) oder Diaza-bicyclo-undecen (DBU).

Bevorzugte Basen für das erfindungsgemäße Verfahren sind NaH, KH, $CaH_2$, $LiNH_2$, $NaNH_2$, $KNH_2$, $Ca(NH_2)_2$, DABCO, DBN, DBU oder eine Stickstoffbase der Formel

$N(R^{19}R^{20}R^{17})$ (VII),

in der

$R^{19}$ und $R^{20}$ gemeinsam $C_4$-$C_8$-Alkylen bedeuten, wobei in der Alkylenkette ein C-Atom durch ein O-Atom oder die Gruppe $-NR^{17}-$ ersetzt sein kann, und

$R^{17}$ Wasserstoff, Methyl oder Ethyl darstellt.

Als Stickstoffbasen der Formel (VI) für den Fall, das $R^9$ und $R^{10}$ gemeinsam $C_4$-$C_8$-Alkylen bedeuten, wobei in der Alkylenkette ein C-Atom durch ein O-Atom oder die Gruppe $-NR^{16}-$ ersetzt sein kann, seien beispielsweise genannt: Pyrrolidin, Piperidin, Piperazin, Morpholin, Pyrrolidin, Aza-cycloheptan, Aza-cyclooctan.

In besonders bevorzugter Weise seien als Basen die Stickstoffbasen der Formel (VII) sowie DABCO, DBN und DBU oder ein Gemisch mehrerer von ihnen genannt.

Die Base wird erfindungsgemäß in einer Menge von 80-600 Mol-%, bevorzugt 95-400 Mol-%, bezogen auf das Arylbromid, eingesetzt.

Das erfindungsgemäße Verfahren wird bei einer Temperatur von 0-160 °C, bevorzugt bei 50-150 °C, durchgeführt.

Für den Fall, daß das einzusetzende Arylbromid nicht flüssig ist, wird im erfindungsgemäßen Verfahren ein Lösungsmittel verwendet. Als Lösungs(Verdünnungs-)mittel kommen in Frage: Kohlenwasserstoffe, insbesondere aromatische Kohlenwasserstoffe, wie Benzol, Toluol oder Xylol; Ether, wie Tetrahydrofuran, Dioxan, Ethylenglykoldimethylether oder Diethylenglykoldimethylether; Alkohole, wie Methanol, Ethanol oder Propanol; chlorierte Kohlenwasserstoffe, wie Chloroform oder Chlorbenzol; Nitrile, wie Acetonitril, Propionitril oder Benzonitril; Amide, wie Dimethylformamid (DMF), N-Methyl-pyrrolidon (NMP) oder Hexamethylphosphorsäuretriamid (HMPT); Sulfoxide, wie Dimethylsulfoxid (DMSO). In bevorzugter Weise werden als Lösungs(Verdünnungs-)mittel eines oder mehrere aus der Gruppe der aromatischen Kohlenwasserstoffe, der Nitrile, der Amide oder der Sulfoxide eingesetzt. Als Menge kommen 80-2000 Gew-%, bevorzugt 100-1000 Gew-%, bezogen auf die Menge des Arylbromids, in Frage. Als Losungs(Verdünnungs-)mittel kommt weiterhin überschüssige Base der obengenannten Art in Frage, sofern diese flüssig ist. In einem solchen Fall wird flüssige Base über die weiter oben in Mol-% angegebene Menge hinaus bis zu der soeben als Gewichtsmenge angegebenen Menge eingesetzt. In vorteilhafter Weise wird ein solches Lösungs-(Verdünnungs-)mittel auch bei flüssigen Arylbromiden eingesetzt.

Das erfindungsgemaße Verfahren ist insbesondere durch den Einsatz einer Intensivbegasungseinrichtung gekennzeichnet, mit Hilfe derer das Acetylen in das flüssige Reaktionsgemisch eingeleitet wird Eine solche Intensivbegasungseinrichtung kann beispielsweise ein Oberflächenbegaser oder ein Volumenbegaser sein, wie ein Kreiselbelüfter, ein Walzenbelüfter, ein Wasserstrahlbelüfter, ein Tauchstrahlbelüfter, ein Rohrrührer, ein Rührer mit separater Gaszufuhr, ein Tauchbelüfter, eine Fritte, ein Lochboden oder ein statischer Mischer oder eine Zweistoffdüse, durch die das Reaktionsgemisch und das Acetylen simultan geführt werden In bevorzugter Weise wird ein Volumenbegaser eingesetzt. In besonders bevorzugter Weise ist dies ein Rohrrührer. Es kann weiterhin günstig sein, die Intensivbegasungseinrichtung durch ein zusätzliches Mischorgan zur Homogenisierung der flüssigen Reaktionsmischung zu ergänzen. Beispielsweise kann ein Rohrrührer mit einem weiteren Rührer, beispielsweise mit einem Propellerrührer, kombiniert werden. Solche Intensivbegasungseinrichtungen und Mischorgane sind dem Fachmann bekannt, beispielsweise aus Ullmann's Encyclopedia of Industrial Chemistry, 5. Aufl., Vol. B2 (1988), 25-21 ff.

Das erfindungsgemäße Verfahren wird im allgemeinen so durchgeführt, daß man das Arylbromid, die Base, den Palladium-Katalysator, sowie gegebenenfalls ein Phosphin der Formel (V) und/oder einen Co-Katalysator vorlegt, wobei bei nicht flüssigen Reaktionskomponenten ein Lösungs(verdünnungs-)mittelmitverwendet wird, und sodann Acetylen mit Hilfe einer Intensivbegasungseinrichtung bei einer Temperatur im genannten Bereich in das Reaktionsgemisch einleitet. Die Reihenfolge der Vorlage der genannten Reaktionsteilnehmer vor Einleitung des Acetylens ist jedoch nicht kritisch und kann auch anders gestaltet werden. Die Reaktion des erfindungsgemäßen Verfahrens ist nicht empfindlich gegenüber Wasser, so das für einen besonderen Wasserausschluß nicht gesorgt zu werden braucht. Es hat sich als günstig erwiesen, die Vorlage der genannten Reaktionsteilnehmer vor dem Einleiten des Acetylens unter einem Schutzgas, wie Stickstoff, vorzunehmen Das Fortschreiten der Reaktion kann in einfacher Weise durch Dünnschichtchromatographie oder Gaschromatographie verfolgt werden. In vielen Fällen fällt das Diaryl-acetylen als Reaktionsprodukt aus dem flüssigen Reaktionsgemisch aus. Weiterhin ist vielfach das aus der Base und dem abgespaltenen Bromwasserstoff gebildete Salz im Reaktionsgemisch nicht löslich. Die Aufarbeitung erfolgt nach üblichen, dem Fachmann geläufigen Methoden Beispielsweise kann ausgefallenes Reaktionsprodukt (Diaryl-acetylen und gegebenenfalls Salz) durch Abdekantieren, Abfiltrieren oder Abzentrifugieren von der flüssigen Phase des Reaktionsgemisches getrennt werden, Das Salz kann sodann durch Behandlung mit Wasser vom Diaryl-acetylen getrennt werden, Das Diaryl-acetylen kann gegebenenfalls durch Umkristallisation oder Säulenchromatographie weiter gereinigt werden, Die abgetrennte flüssige Phase des Reaktionsgemisches kann für einen nächsten Ansatz wiederverwendet werden. Man kann jedoch auch durch Destillation der abgetrennten flüssigen Phase das Lösungs(Verdünnungs-)mittel und die katalytisch wirkenden Bestandteile trennen und getrennt recyclisieren und wieder aufarbeiten.

Das erfindungsgemäße Verfahren ermöglicht die Herstellung von symmetrischen Diaryl-acetylenen in hoher Ausbeute und hoher Selektivität in nur einer Reaktionsstufe, ausgehend von gut zugänglichen Arylbromiden, und ist somit den bisher bekannten Verfahren in überzeugender Weise überlegen.

Beispiele

Beispiel 1

4,4'-Bis-(carboethoxy)tolan

Unter Stickstoff legte man 343,5 g 4-Brombenzoesäureethylester, 10,5 g $(P\text{-phenyl}_3)_2.PdCl_2$, 10,5 g Kupfer(I)iodid, 63 g Triphenylphosphin und 600 ml Piperidin in 600 ml Acetonitril vor Während man dann über einen Rohrrührer Acetylen in das Reaktionsgemisch einleitete, wurde die Temperatur auf ca. 80 °C erhöht Die Acetyleneinleitung wurde so lange fortgesetzt, bis der 4-Brombenzoesäureethylester vollständig umgesetzt war. Dann ließ man das Reaktionsgemisch abkühlen und saugte das auskristallisierte Reaktionsprodukt ab Man erhielt 221 g (91 %) 4,4'-Bis-(carboethoxy)tolan von Fp. 151-153 °C. Durch Umkristallisation (z.B. aus Toluol) oder durch Filtration über Kieselgel kann das Produkt weiter gereinigt werden,

Die Beispiele 2-15 beschreiben die Herstellung verschiedener Diarylacetylene mit Hilfe des erfindungsgemäßen Verfahrens analog Beispiel 1.

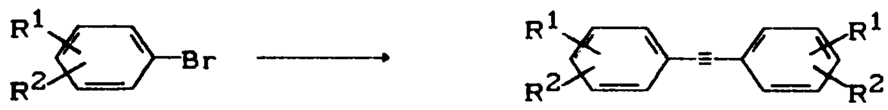

| Nr. | $R^1$ | $R^2$ | Base[1)] | Fp [°C] | Diarylacetylen Ausbeute [%] |
|---|---|---|---|---|---|
| 2 [2)] | H | p-$COOC_2H_5$ | Piperidin | 151-153 | 86 |
| 3 | H | p-$COOC_2H_5$ | Pyrrolidin | 151-153 | 79 |
| 4 | H | p-$COOC_2H_5$ | Aza-cyclohep-tan | 151-153 | 83 |
| 5 | H | p-$COOC_2H_5$ | DBU | 151-153 | 67 |
| 6 | H | p-$COOC_2H_5$ | DBN | 151-153 | 55 |
| 7 | H | p-$COOC_2H_5$ | DABCO | 151-153 | 59 |
| 8 | H | p-$COOtC_4H_9$ | Piperidin | | 90 |
| 9 | m-$COOC_2H_5$ | H | Piperidin | 84-89 | 73 |
| 10 | H | p-$\overset{O}{\overset{\|}{C}}CH_3$ | Piperidin | 195-196 | quant. |
| 11 | m-$COOCH_3$ | p-$COOCH_3$ | | 130-132 | 81 |

| Nr. | $R^1$ | $R^2$ | Base 1) | Fp [°C] | Diarylacetylen Ausbeute [%] |
|---|---|---|---|---|---|
| 12 | H | p-$C_6H_5$ | Piperidin | 236-237 | 87 |
| 13 | H | 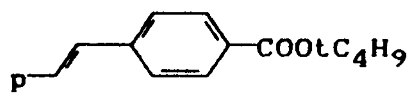 | Piperidin | ›280° C 3) | 98 |
| 14 | H | 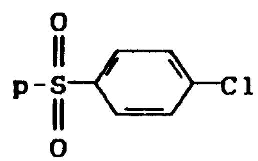 | Piperidin | ›280° C 3) | 87 |
| 15 | H | 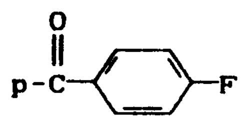 | Piperidin | 222-224 | 64 |
| 16 | H | 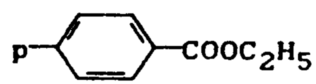 | Piperidin | ›280° C 3) | 88 |

1) 0,8 mol bezogen auf 0,2 mol eingesetztes Arylbromid

2) Cu(II)acetat-Monohydrat anstelle von Cu(I)iodid

3) massenspektrometrisch charakterisiert

## Patentansprüche

**1.** Verfahren zur Herstellung von symmetrischen Diarylacetylenen der Formel

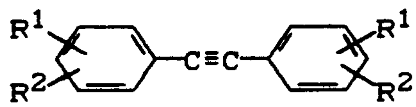

,

in der

$R^1$ Wasserstoff, geradkettiges oder verzweigtes $C_1$-$C_8$-Alkyl, $C_3$-$C_8$-Cycloalkyl, Phenyl, $C_7$-$C_{10}$-Aralkyl geradkettiges oder verzweigtes $C_1$-$C_8$-Alkoxy, Phenoxy, Benzyloxy, $\beta$-Phenyl-ethenyl, Phenyl-ethinyl, Phenyl-sulfonyl, Phenyl-carbonyl, Hydroxy in freier oder geschützter Form, Fluor, Chlor, Nitro, Cyano, $COR^3$, $NR^4R^5$ oder $CONR^4R^5$ bedeutet und

$R^2$ für Wasserstoff, geradkettiges oder verzweigtes $C_1$-$C_8$-Alkyl, Fluor, Chlor, Cyano, $COR^3$ oder $CONR^4R^5$ steht und

$R^1$ und $R^2$ gemeinsam, wenn sie benachbart sind, eine Dicarbonsäureanhydridgruppe bilden können,

wobei

$R^3$ Wasserstoff, Hydroxy, geradkettiges oder verzweigtes $C_1$-$C_8$-Alkyl, geradkettiges oder verzweigtes $C_1$-$C_8$-Alkoxy, Phenyl oder Phenoxy bedeutet und

$R^4$ und $R^5$ unabhängig voneinander für Wasserstoff oder geradkettiges oder verzweigtes $C_1$-$C_8$-Alkyl stehen und $R^4$ zusätzlich $C_2$-$C_4$-Alkoxy-carbonyl oder $C_2$-$C_4$-Acyl sein kann, wobei weiterhin $R^4$ und $R^5$ gemeinsam $C_4$-$C_8$-Alkylen bilden können,

und

wobei die Benzolkerne in den Resten $R^1$ und $R^2$ ihrerseits durch geradkettiges oder verzweigtes $C_1$-$C_8$-Alkyl, geradkettiges oder verzweigtes $C_1$-$C_8$-Alkoxy, Fluor, Chlor oder $COR^3$ substituiert sein können,

durch Umsetzung eines Arylhalogenids mit Acetylen in Gegenwart eines Palladium-Katalysators und einer Base, dadurch gekennzeichnet, das man das Acetylen mit Hilfe einer Intensivbegasungseinrichtung in das flüssige Reaktionsgemisch einleitet und als Arylhalogenide Arylbromide der Formel

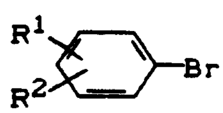

einsetzt, in der

$R^1$ und $R^2$ die obige Bedeutung haben.

**2.** Verfahren nach Anspruch 1, dadurch gekennzeichnet, daß als Arylbromid ein solches der Formel

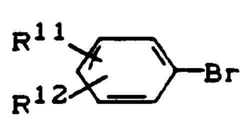

eingesetzt wird, in der

$R^{11}$ Wasserstoff, geradkettiges oder verzweigtes $C_1$-$C_8$-Alkyl, Phenyl, Benzyl, geradkettiges oder verzweigtes $C_1$-$C_8$-Alkoxy, Phenoxy, Benzyloxy, $\beta$-Phenyl-ethenyl, Phenylsulfonyl, Phenyl-carbonyl, Fluor, Chlor, Nitro, Cyano, $COR^{13}$ oder $NR^{14}R^{15}$ bedeutet und

$R^{12}$ für Wasserstoff, geradkettiges oder verzweigtes $C_1$-$C_8$-Alkyl, Fluor, Chlor, Cyano oder $COR^{13}$ steht und

$R^{11}$ und $R^{12}$ gemeinsam, wenn sie benachbart sind, eine Dicarbonsäureanhydridgruppe bilden können,
wobei
$R^{13}$ Wasserstoff, Hydroxy, geradkettiges oder verzweigtes $C_1$-$C_4$-Alkyl oder geradkettiges oder verzweigtes $C_1$-$C_4$-Alkoxy bedeutet und
$R^{14}$ und $R^{15}$ unabhängig voneinander für Wasserstoff oder geradkettiges oder verzweigtes $C_1$-$C_4$-Alkyl stehen und $R^{14}$ zusätzlich $C_2$-$C_4$-Acyl sein kann, wobei weiterhin $R^{14}$ und $R^{15}$ gemeinsam $C_4$-$C_8$-Alkylen bilden können,
und
wobei die Benzolkerne in den Resten $R^{11}$ und $R^{12}$ ihrerseits durch geradkettiges oder verzweigtes $C_1$-$C_4$-Alkyl, geradkettiges oder verzweigtes $C_1$-$C_4$-Alkoxy, Fluor, Chlor oder $COR^{13}$ substituiert sein können,

**3.** Verfahren nach Anspruch 2, dadurch gekennzeichnet, daß als Arylbromid ein solches der Formel

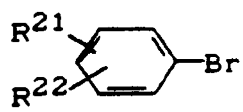

eingesetzt wird, in der
$R^{21}$ Wasserstoff, geradkettiges oder verzweigtes $C_1$-$C_4$-Alkyl, Phenyl, $\beta$-Phenyl-ethenyl, Phenyl-sulfonyl, Phenyl-carbonyl, Fluor, Chlor, Cyano oder $COR^{23}$ bedeutet und
$R^{22}$ für Wasserstoff, Methyl, Ethyl, Cyano oder $COR^{23}$ steht und
$R^{21}$ und $R^{22}$ gemeinsam, wenn sie benachbart sind, eine Dicarbonsäuregruppe bilden können,
wobei
$R^{23}$ Wasserstoff, Hydroxy, Methyl, Ethyl, Methoxy oder Ethoxy bedeutet und wobei die Benzolkerne im Rest $R^{21}$ ihrerseits durch Fluor, Chlor oder $COR^{23}$ substituiert sein können.

**4.** Verfahren nach Anspruch 1, dadurch gekennzeichnet, das als Palladium-Katalysator ein oder mehrere Stoffe aus der Gruppe Palladium-Schwarz, Palladium/Kohle, einfache anorganische und organische Palladium-Salze, komplexierte anorganische und organische Palladium-Salze, wobei als Komplexbildner Benzonitril, $C_1$-$C_4$-Alkylnitril, Triphenylarsin oder ein Phosphin der Formel

$P(R^6R^7R^8)$ ,

in der
$R^6$, $R^7$ und $R^8$ unabhängig voneinander geradkettiges oder verzweigtes $C_1$-$C_4$-Alkyl oder Phenyl ist, wobei Phenyl durch geradkettiges oder verzweigtes $C_1$-$C_4$-Alkyl oder $C_1$-$C_4$-Alkoxy oder durch Fluor, Chlor oder Brom substituiert sein kann,
fungiert, Phenyl-palladium-iodid-diphosphin oder Palladium-tetraphosphin, wobei in den beiden letzten Fällen ein Phosphin der genannten Art auftritt, Di-(dibenzyliden-aceton)-palladium oder Di-(1,1-bis-[diphenylphosphin]-ferrocen)-palladiumdichlorid, -dibromid oder -diiodid, bevorzugt organische Palladium-Salze, komplexierte anorganische und organische Palladium-Salze, wobei als Komplexbildner ein Phosphin der genannten Art fungiert, und Palladium-tetraphosphin mit einem Phosphin der genannten Art in einer Menge von 0,0005-5 Mol-%, bevorzugt 0,05-2 Mol-%, bezogen auf das Arylbromid, eingesetzt wird (werden).

**5.** Verfahren nach Anspruch 1, dadurch gekennzeichnet, das der Palladium-Katalysator durch Zusatz eines oder mehrerer Phosphins (Phosphine) der Formel

$P(R^6R^7R^8)$ ,

in der
$R^6$, $R^7$ und $R^8$ unabhängig voneinander geradkettiges oder verzweigtes $C_1$-$C_4$-Alkyl oder Phenyl ist, wobei Phenyl durch geradkettiges oder verzweigtes $C_1$-$C_4$-Alkyl oder $C_1$-$C_4$-

Alkoxy oder durch Fluor, Chlor oder Brom substituiert sein kann,
in einer Menge von 100-2000 Mol-%, bezogen auf den Palladium-Katalysator, aktiviert wird.

**6.** Verfahren nach Anspruch 1, dadurch gekennzeichnet, das dem Palladium-Katalysator ein oder mehrere Kupfersalz(e) als Co-Katalysatoren, bevorzugt Cu-halogenid oder Cu-acetat, besonders bevorzugt Cu(I)-iodid oder Cu(II)acetat-Monohydrat, in einer Menge von 50-1000 Mol-%, bezogen auf den Palladium-Katalysator, zugesetzt wird (werden).

**7.** Verfahren nach Anspruch 1, dadurch gekennzeichnet, daß als Base eine oder mehrere Verbindungen aus der Gruppe der Hydride, Hydroxide, Oxide oder Amide der Alkalimetalle oder Erdalkalimetalle, der geradkettigen oder verzweigten $C_1$-$C_8$-Alkoholate der Alkalimetalle oder Erdalkalimetalle, der Stickstoffbasen der Formel

$N(R^9R^{10}R^{16})$ ,

in der

$R^9$ und $R^{10}$ unabhängig voneinander geradkettiges oder verzeigtes $C_1$-$C_8$-Alkyl oder beide gemeinsam $C_4$-$C_8$-Alkylen bedeuten, wobei in der Alkylenkette ein C-Atom durch ein O-Atom oder die Gruppe $-NR^{16}-$ ersetzt sein kann, und

$R^{16}$ Wasserstoff oder geradkettiges oder verzweigtes $C_1$-$C_4$-Alkyl darstellt,

oder Triethylendiamin, Diaza-bicyclo-nonen oder Diaza-bicyclo-undecen,
bevorzugt eine oder mehrere aus der Gruppe NaH, KH, $CaH_2$, $LiNH_2$, $NaNH_2$, $KNH_2$, $Ca(NH_2)_2$, der Stickstoffbasen der Formel

$N(R^{19}R^{20}R^{17})$ ,

in der

$R^{19}$ und $R^{20}$ gemeinsam $C_4$-$C_8$-Alkylen bedeuten, wobei in der Alkylenkette ein C-Atom durch ein O-Atom oder die Gruppe $-NR^{17}-$ ersetzt sein kann, und

$R^{17}$ Wasserstoff, Methyl oder Ethyl darstellt,

oder Triethylendiamin, Diaza-bicyclo-nonen oder Diaza-bicyclo-undecen,
besonders bevorzugt eine oder mehrere aus der Gruppe der Stickstoffbasen der Formel $N(R^{19}R^{20}R^{17})$-,Triethylendiamin, Diaza-bicyclo-nonen und Diaza-bicyclo-undecen
in einer Menge von 80-600 Mol-%, bevorzugt 95-400 Mol-% bezogen auf das Arylbromid, eingesetzt wird (werden).

**8.** Verfahren nach Anspruch 1, dadurch gekennzeichnet, das bei einer Temperatur von 0-160 °C, bevorzugt 50-150 °C gearbeitet wird.

**9.** Verfahren nach Anspruch 1, dadurch gekennzeichnet, das in einem oder mehreren Lösungs-(verdünnungs-)mitteln in einer Menge von 80-2000 Gew.-%, bevorzugt 100-1000 Gew.-%, bezogen auf die Menge des Arylbromids, gearbeitet wird, wobei das oder die Lösungs(verdünnungs-)mittel aus der Gruppe der Kohlenwasserstoffe, der Ether, der Alkohole, der chlorierten Kohlenwasserstoffe, der Nitrile, der Amide oder Sulfoxide stammt und wobei weiterhin überschüssige Base, sofern diese flüssig ist, als Lösungs(verdünnungs-)mittel dienen kann.

**10.** Verfahren nach Anspruch 1, dadurch gekennzeichnet, daß als Intensivbegasungseinrichtung für das Acetylen ein Oberflächenbegaser oder ein Volumenbegaser, bevorzugt ein Volumenbegaser, besonders bevorzugt ein Rohrrührer eingesetzt wird, wobei die Reaktionsmischung durch ein zusätzliches Mischorgan homogenisiert werden kann.

## Claims

1. Process for the preparation of symmetrical diarylacetylenes of the formula

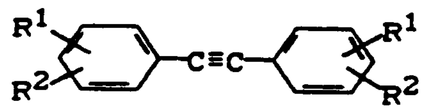

,

in which

| | |
|---|---|
| $R^1$ | is hydrogen, straight-chain or branched $C_1$-$C_8$-alkyl, $C_3$-$C_8$-cycloalkyl, phenyl, $C_7$-$C_{10}$-aralkyl, straight-chain or branched $C_1$-$C_8$-alkoxy, phenoxy, benzyloxy, $\beta$-phenyl-ethenyl, phenyl-ethinyl, phenyl-sulphonyl, phenyl-carbonyl, hydroxyl in free or protected form, fluorine, chlorine, nitro, cyano, $COR^3$, $NR^4R^5$ or $CONR^4R^5$ and |
| $R^2$ | represents hydrogen, straight-chain or branched $C_1$-$C_8$-alkyl, fluorine, chlorine, cyano, $COR^3$ or $CONR^4R^5$ and |
| $R^1$ and $R^2$, | if they are adjacent, may together form a dicarboxylic anhydride group, where |
| $R^3$ | is hydrogen, hydroxyl, straight-chain or branched $C_1$-$C_8$-alkyl, straight-chain or branched $C_1$-$C_8$-alkoxy, phenyl or phenoxy and |
| $R^4$ and $R^5$, | independently of one another, represent hydrogen or straight-chain or branched $C_1$-$C_8$-alkyl and $R^4$ may also be $C_2$-$C_4$-alkoxycarbonyl or $C_2$-$C_4$-acyl, and furthermore $R^4$ and $R^5$ may together form $C_4$-$C_8$-alkylene, and it being possible for the benzene rings in the radicals $R^1$ and $R^2$ for their part to be substituted by straight-chain or branched $C_1$-$C_8$-alkyl, straight-chain or branched $C_1$-$C_8$-alkoxy, fluorine, chlorine or $COR^3$, |

by reaction of an aryl halide with acetylene in the presence of a palladium catalyst and a base, characterized in that the acetylene is introduced into the liquid reaction mixture using a high intensity gas dispersion means and the aryl halides used are aryl bromides of the formula

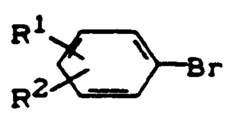

in which
$R^1$ and $R^2$ are as defined above.

2. Process according to Claim 1, characterized in that the aryl bromide used is one of the formula

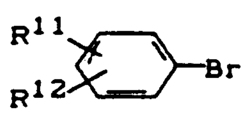

in which

| | |
|---|---|
| $R^{11}$ | is hydrogen, straight-chain or branched $C_1$-$C_8$-alkyl, phenyl, benzyl, straight-chain or branched $C_1$-$C_8$-alkoxy, phenoxy, benzyloxy, $\beta$-phenyl-ethenyl, phenyl-sulphonyl, phenyl-carbonyl, fluorine, chlorine, nitro, cyano, $COR^{13}$ or $NR^{14}R^{15}$ and |
| $R^{12}$ | represents hydrogen, straight-chain or branched $C_1$-$C_8$-alkyl, fluorine, chlorine, cyano or $COR^{13}$ and |
| $R^{11}$ and $R^{12}$, | if they are adjacent, may together form a dicarboxylic anhydride group, where |
| $R^{13}$ | is hydrogen, hydroxyl, straight-chain or branched $C_1$-$C_4$-alkyl or straight-chain or |

branched $C_1$-$C_4$-alkoxy and

$R^{14}$ and $R^{15}$, independently of one another, represent hydrogen or straight-chain or branched $C_1$-$C_4$-alkyl and $R^{14}$ may also be $C_2$-$C_4$-acyl and, moreover, $R^{14}$ and $R^{15}$ may together form $C_4$-$C_8$-alkylene,

and

it being possible for the benzene rings in the radicals $R^{11}$ and $R^{12}$ for their part to be substituted by straight-chain or branched $C_1$-$C_4$-alkyl, straight-chain or branched $C_1$-$C_4$-alkoxy, fluorine, chlorine or $COR^{13}$.

3. Process according to Claim 2, characterized in that the aryl bromide used is one of the formula

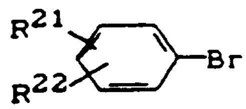

in which

$R^{21}$ is hydrogen, straight-chain or branched $C_1$-$C_4$-alkyl, phenyl, $\beta$-phenyl-ethenyl, phenyl-sulphonyl, phenyl-carbonyl, fluorine, chlorine, cyano or $COR^{23}$ and

$R^{22}$ represents hydrogen, methyl, ethyl, cyano or $COR^{23}$ and

$R^{21}$ and $R^{22}$, if they are adjacent, may together form a dicarboxylic anhydride group,

where

$R^{23}$ is hydrogen, hydroxyl, methyl, ethyl, methoxy or ethoxy and it being possible for the benzene rings in the radical $R^{21}$ for their part to be substituted by fluorine, chlorine or $COR^{23}$.

4. Process according to Claim 1, characterized in that the palladium catalyst used comprises one or more substances from the group consisting of palladium black, palladium/carbon, simple inorganic and organic palladium salts, complexed inorganic and organic palladium salts in which the complexing agent is benzonitrile, $C_1$-$C_4$-alkyl nitrile, triphenylarsine or a phosphine of the formula

$P(R^6R^7R^8)$

in which

$R^6$, $R^7$ and $R^8$, independently of one another, are straight-chain or branched $C_1$-$C_4$-alkyl or phenyl, it being possible for phenyl to be substituted by straight-chain or branched $C_1$-$C_4$-alkylor $C_1$-$C_4$-alkoxy or by fluorine, chlorine or bromine, phenyl-palladium-diphosphine iodide or palladium-tetraphosphine, in the last two cases a phosphine of the abovementioned type being used, di-(dibenzylidene-acetone)-palladium or di-(1,1-bis[diphenyl-phosphine]-ferrocene)palladiumdichloride, dibromide or diiodide,

preferably organic palladium salts, complexed inorganic and organic palladium salts in which the complexing agent is a phosphine of the above-mentioned type, and palladium-tetraphosphine containing a phosphine of the abovementioned type, in an amount of 0.0005-5 mol%, preferably 0.05-2 mol%, relative to the aryl bromide.

5. Process according to Claim 1, characterized in that the palladium catalyst is activated by the addition of one or more phosphines of the formula

$P(R^6R^7R^8)$

in which

$R^6$, $R^7$ and $R^8$, independently of one another, are straight-chain or branched $C_1$-$C_4$-alkyl or phenyl, it being possible for phenyl to be substituted by straight-chain or branched $C_1$-$C_4$-alkyl or $C_1$-$C_4$-alkoxy or by fluorine, chlorine or bromine,

in an amount of 100-2000 mol%, relative to the palladium catalyst.

6. Process according to Claim 1, characterized in that the palladium catalyst has added to it one or more copper salts as cocatalysts, preferably Cu halide or Cu acetate, particularly preferably Cu(I) iodide or Cu(II) acetate monohydrate, in an amount of 50-1000 mol%, relative to the palladium catalyst.

7. Process according to Claim 1, characterized in that the base used is one or more compounds from the group of the hydrides, hydroxides, oxides or amides of the alkali metals or alkaline earth metals, the straight-chain or branched $C_1$-$C_8$-alcoholates of the alkali metals or alkaline earth metals, the nitrogen bases of the formula

$N(R^9R^{10}R^{16})$

in which

$R^9$ and $R^{10}$, independently of one another, are straight-chain or branched $C_1$-$C_8$-alkyl or else the two together are $C_4$-$C_8$-alkylene, it being possible in the alkylene chain for one carbon atom to be replaced by an oxygen atom or the group $-NR^{16}-$, and

$R^{16}$ is hydrogen or straight-chain or branched $C_1$-$C_4$-alkyl,

or triethylenediamine, diaza-bicyclo-nonene or diaza-bicyclo-undecene,
preferably one or more from the group consisting of NaH, KH, $CaH_2$, $LiNH_2$, $NaNH_2$, $KNH_2$, $Ca(NH_2)_2$, the nitrogen bases of the formula

$N(R^{19}R^{20}R^{17})$

in which

$R^{19}$ and $R^{20}$ together are $C_4$-$C_8$-alkylene, it being possible in the alkylene chain for one carbon atom to be replaced by an oxygen atom or the group $-NR^{17}-$, and

$R^{17}$ is hydrogen, methyl or ethyl,

or triethylene diamine, diaza-bicyclo-nonene or diaza-bicyclo-undecene,
particularly preferably one or more from the group of the nitrogen bases of the formula $N(R^{19}R^{20}R^{17})$-,triethylenediamine, diaza-bicyclo-nonene and diazabicyclo-undecene,
in an amount of 80-600 mol%, preferably 95-400 mol%, relative to the aryl bromide.

8. Process according to Claim 1, characterized in that it is carried out at 0-160 °C, preferably 50-150 °C.

9. Process according to Claim 1, characterized in that it is carried out in one or more solvents (diluents) present in an amount of 80-2000 % by weight, preferably 100-1000 % by weight, relative to the amount of aryl bromide the solvent (diluent) being selected from the group of the hydrocarbons, ethers, alcohols, chlorinated hydrocarbons, nitriles, amides and sulphoxides, and it furthermore being possible to use excess base, as long as this is a liquid, as the solvent (diluent).

10. Process according to Claim 1, characterized in that the high intensity gas dispersion means used for the acetylene is a surface aerator or a volume aerator, preferably a volume aerator, particularly preferably a tubular stirrer, it being possible to use an additional mixer to homogenize the reaction mixture.

## Revendications

1. Procédé de préparation de diarylacétylènes symétriques de formule

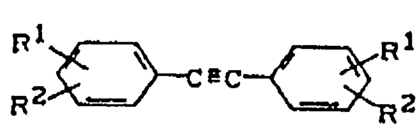

dans laquelle

$R^1$ désigne l'hydrogène, un radical alkyle $C_1$-$C_8$ à chaîne droite ou ramifiée, un radical cycloalkyle $C_3$-$C_8$, un radical phényle, un radical aralkyle $C_7$-$C_{10}$, un radical alcoxy $C_1$-$C_8$ à chaîne droite ou ramifiée, un radical phénoxy, benzyloxy, $\beta$-phényléthényle,

phényléthynyle, phénylsulfonyle, phénylcarbonyle, un radical hydroxy sous forme libre ou protégée, le fluor, le chlore, un radical nitro, cyano, $COR^3$, $NR^4R^5$ ou $CONR^4R^5$, et

$R^2$ désigne l'hydrogène, un radical alkyle $C_1$-$C_8$ à chaîne droite ou ramifiée, le fluor, le chlore, un radical cyano, $COR^3$ ou $CONR^4R^5$, et

$R^1$ et $R^2$ peuvent former ensemble, lorsqu'ils sont voisins, un radical anhydride d'acide dicarboxylique,

où

$R^3$ désigne l'hydrogène, un radical hydroxy, un radical alkyle $C_1$-$C_8$ à chaîne droite ou ramifiée, un radical alcoxy $C_1$-$C_8$ à chaîne droite ou ramifiée, un radical phényle ou phénoxy, et

$R^4$ et $R^5$ désignent, indépendamment l'un de l'autre, l'hydrogène ou un radical alkyle $C_1$-$C_8$ à chaîne droite ou ramifiée et $R^4$ peut par ailleurs être un radical alcoxycarbonyle $C_2$-$C_4$ ou acyle $C_2$-$C_4$, $R^4$ et $R^5$ pouvant par ailleurs former ensemble un radical alkylène $C_4$-$C_8$,

et

les noyaux benzéniques dans les radicaux $R^1$ et $R^2$ peuvent quant à eux être substitués par un radical alkyle $C_1$-$C_8$ à chaîne droite ou ramifiée, un radical alcoxy $C_1$-$C_8$ à chaîne droite ou ramifiée, la fluor, le chlore ou un radical $COR^3$,

par réaction d'un halogénure d'aryle avec de l'acétylène en présence d'un catalyseur de palladium et d'une base, caractérisé en ce que l'acétylène est introduit dans le mélange réactif liquide à l'aide d'un dispositif de gazéification intensive et en ce que l'halogénure d'aryle utilisé est un bromure d'aryle de formule

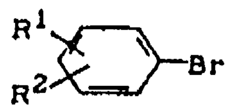

dans laquelle
$R^1$ et $R^2$ ont la signification précitée.

2. Procédé selon la revendication 1, caractérisé en ce que le bromure d'aryle utilisé répond à la formule:

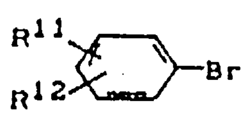

dans laquelle

$R^{11}$ désigne l'hydrogène, un radical alkyle $C_1$-$C_8$ à chaîne droite ou ramifiée, un radical phényle, benzyle, un radical alcoxy $C_1$-$C_8$ à chaîne droite ou ramifiée, un radical phénoxy, benzyloxy, $\beta$-phényléthényle, phénylsulfonyle, phénylcarbonyle, le fluor, le chlore, un radical nitro, cyano, $COR^{13}$ ou $NR^{14}R^{15}$, et

$R^{12}$ désigne l'hydrogène, un radical alkyle $C_1$-$C_8$ à chaîne droite ou ramifiée, le fluor, le chlore, un radical cyano ou $COR^{13}$, et

$R^{11}$ et $R^{12}$ peuvent former ensemble, lorsqu'ils sont voisins, un radical anhydride d'acide dicarboxylique,

où

$R^{13}$ désigne l'hydrogène, un radical hydroxy, un radical alkyle $C_1$-$C_4$ à chaîne droite ou ramifiée ou un radical alcoxy $C_1$-$C_4$ à chaîne droite ou ramifiée, et

$R^{14}$ et $R^{15}$ désignent, indépendamment l'un de l'autre, l'hydrogène ou un radical alkyle $C_1$-$C_4$ à chaîne droite ou ramifiée et $R^{14}$ peut également être un radical acyle $C_2$-$C_4$, $R^{14}$ et $R^{15}$ pouvant par ailleurs former ensemble un radical alkylène $C_4$-$C_8$,

et

les noyaux benzéniques dans les radicaux $R^{11}$ et $R^{12}$ peuvent quant à eux être substitués par un radical alkyle $C_1$-$C_4$ à chaîne droite ou ramifiée, un radical alcoxy

$C_1$-$C_4$ à chaîne droite ou ramifiée, le fluor, le chlore ou un radical $COR^{13}$.

**3.** Procédé selon la revendication 2, caractérisé en ce que le bromure d'aryle utilisé répond à la formule

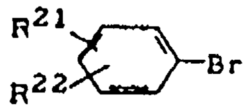

dans laquelle

- $R^{21}$ désigne l'hydrogène, un radical alkyle $C_1$-$C_4$ à chaîne droite ou ramifiée, un radical phényle, $\beta$-phényléthényle, phénylsulfonyle, phénylcarbonyle, le fluor, le chlore, un radical cyano ou $COR^{23}$, et
- $R^{22}$ désigne l'hydrogène, un radical méthyle, éthyle, cyano ou $COR^{23}$, et
- $R^{21}$ et $R^{22}$ peuvent former ensemble, lorsqu'ils sont voisins, un radical anhydride d'acide dicarboxylique,
  où
- $R^{23}$ désigne l'hydrogène, un radical hydroxy, méthyle, éthyle, méthoxy ou éthoxy et les noyaux benzéniques dans le radical $R^{23}$ peuvent quant à eux être substitués par le fluor, le chlore ou un radical $COR^{23}$.

**4.** Procédé selon la revendication 1, caractérisé en ce que le catalyseur de palladium peut être une ou plusieurs des substances du groupe composé du noir de palladium, du palladium sur carbone, de sels de palladium inorganiques et organiques simples, de sels de palladium inorganiques et organiques complexés, l'agent complexant mis en oeuvre étant le benzonitrile, un alkylnitrile $C_1$-$C_4$, la triphénylarsine ou une phosphine de formule

$P(R^6 R^7 R^8)$

dans laquelle

- $R^6$, $R^7$ et $R^8$ désignant, indépendamment l'un de l'autre, un radical alkyle $C_1$-$C_4$ à chaîne droite ou ramifiée ou un radical phényle, le radical phényle pouvant être substitué par un radical alkyle $C_1$-$C_4$ à chaîne droite ou ramifiée, ou un radical alcoxy $C_1$-$C_4$ ou par le fluor, le chlore ou le brome,

la diphosphine d'iodure de phénylpalladium ou la tétraphosphine de palladium, une phosphine du type précité se rencontrant dans les deux derniers cas, le di-(di-benzylidènacétone)-palladium ou le dichlorure, le dibromure ou le diiodure de di-(1,1-bis[diphénylphosphine]-ferrocène)-palladium, de préférence des sels de palladium organiques, des sels de palladium inorganiques et orgamiques complexés, pour lesquels l'agent complexant utilisé est une phosphine du type précité, et la tétraphosphine de palladium avec une phosphine du type précité, dans une quantité de 0,0005-5 % en moles, de préférence 0,05 à 2 % en moles par rapport au bromure d'aryle.

**5.** Procédé selon la revendication 1, caractérisé en ce que le catalyseur de palladium est activé par l'addition d'une ou plusieurs phosphines de formule

$P(R^6 R^7 R^8)$

dans laquelle

- $R^6$, $R^7$ et $R^8$ désignent, indépendamment l'un de l'autre, un radical alkyle $C_1$-$C_4$ à chaîne droite ou ramifiée ou un radical phényle, le radical phényle pouvant être substitué par un radical alkyle $C_1$-$C_4$ à chaîne droite ou ramifiée, ou un radical alcoxy $C_1$-$C_4$ ou par le fluor, le chlore ou le brome,

dans une quantité de 100 à 2000 % en moles par rapport au catalyseur de palladium.

**6.** Procédé selon la revendication 1, caractérisé en ce qu'un ou plusieurs sels de cuivre sont ajoutés au catalyseur de palladium, à titre de cocatalyseurs, de préférence un halogénure de cuivre ou un acétate de cuivre, en particulier l'iodure de Cu(I) ou le monohydrate d'acétate de Cu(II) dans une quantité de 50

à 1000 % en moles par rapport au catalyseur de palladium.

**7.** Procédé selon la revendication 1, caractérisé en ce qu'un ou plusieurs des composés du groupe des hydrures, hydroxydes, oxydes ou amides de métaux alcalins ou alcalino-terreux, des alcoolates $C_1$-$C_8$ à chaîne droite ou ramifiée de métaux alcalins ou alcalino-terreux, des bases azotées, de formule

$N(R^9R^{10}R^{16})$

dans laquelle

$R^9$ et $R^{10}$ désignent, indépendamment l'un de l'autre, un radical alkyle $C_1$-$C_8$ à chaîne droite ou ramifiée ou tous deux désignent ensemble un radical alkylène $C_4$-$C_8$, un atome C pouvant être remplacé dans la chaîne alkylène par un atome O ou le radical $-NR^{16}-$, et

$R^{16}$ désigne l'hydrogène ou un radical alkyle $C_1$-$C_4$ à chaîne droite ou ramifiée,

ou la triéthylènediamine, le diazabicyclononène ou le diazabicycloundécène,

de préférence un ou plusieurs composés du groupe NaH, KH, $CaH_2$, $LiNH_2$, $NaNH_2$, $KNH_2$, $Ca(NH_2)_2$, les bases azotées de formule

$N(R^{19}R^{20}R^{17})$

dans laquelle

$R^{19}$ et $R^{2\circ}$ désignent ensemble un radical alkylène $C_4$-$C_8$, un atome C dans la chaîne alkylène pouvant être remplacé par un atome O ou le radical $-NR^{17}-$, et

$R^{17}$ représente l'hydrogène, un radical méthyle ou éthyle,

ou la triéthylènediamine, le diazabicyclononène ou le diazabicycloundécène,

tout particulièrement un ou plusieurs des composés du groupe des bases azotées de formule $N(R^{19}R^{20}R^{17})$, la triéthylènediamine, le diazabicyclononène et le diazabicycloundécène

sont utilisés comme base dans une quantité de 80 à 600 % en moles, de préférence 95 à 400 % en moles par rapport au bromure d'aryle.

**8.** Procédé selon la revendication 1, caractérisé en ce que la réaction est menée à une température de 0 à 160 °C, de préférence de 50 à 150 °C.

**9.** Procédé selon la revendication 1, caractérisé en ce que la réaction se déroule dans un ou plusieurs solvants(diluants) dans une quantité de 80 à 2000 % en poids, de préférence 100 à 1000 % en poids par rapport à la quantité de bromure d'aryle, le ou les solvants(diluants) provenant du groupe des hydrocarbures, des nitriles, des amides ou sulfoxydes et une base excédentaire, pour autant que celle-ci soit liquide, pouvant servir de solvant (diluant).

**10.** Procédé selon la revendication 1 caractérisé en ce que le dispositif de gazéification intensive pour l'acétylène est un gazéificateur en surface ou un gazéificateur de volume, de préférence un gazéificateur de volume, tout particulièrement un agitateur tubulaire, le mélange réactif pouvant être homogénéisé par un organe mélangeur supplémentaire.